# EUROPEAN PATENT SPECIFICATION

(11) **EP 1 945 279 B1**
(45) Date of publication and mention of the grant of the patent: **27.06.2012**
(21) Application number: 06814838.6
(22) Date of filing: 18.09.2006
(51) Int. Cl.: A61L 27/34, A61L 29/08, A61L 31/10

(54) **INTERNAL MEDICAL DEVICES HAVING POLYELECTROLYTE-CONTAINING EXTRUDED REGIONS**
INNERES MEDIZINPRODUKT MIT POLYELEKTROLYT-HALTIGEN EXTRUDIERTEN REGIONEN
DISPOSITIFS MEDICAUX INTERNES PRESENTANT DES ZONES EXTRUDEES CONTENANT UN POLYELECTROLYTE

(30) Priority: 21.09.2005 US 231583
(43) Date of publication of application: 23.07.2008
(73) Proprietor: Boston Scientific Limited, Barbados, West Indies (BB)
(72) Inventor: ATANASOSKA, Liliana, Edina, MN 55436 (US); WEBER, Jan, Maple Grove, MN 55125 (US); WARNER, Robert, Woodbury, MN 55311 (US)
(74) Representative: Hermann, Gerhard
(86) International application number: PCT/US2006/036245
(87) International publication number: WO 2007/038043

(56) References cited:
- US-A1- 2003 157 260
- US-A1- 2004 249 469
- US-A1- 2005 096 509
- GREGORY M GRATSON, MINGJIE XU, JENNIFER A. LEWIS: "Direct writing of three-dimensional webs" NATURE, vol. 428, 25 March 2004 (2004-03-25), page 386, XP002469278 cited in the application
- GREGORY M. GRATSON, JENNIFER A. LEWIS: "Phase Behavior and Rheological Properties of Polyelectrolyte Inks for Direct-Write Asssembly" LANGMUIR, vol. 21, 4 January 2005 (2005-01-04), pages 457-464, XP002469279 cited in the application
- LEWIS J A ET AL: "Direct writing in three dimensions" MATERIALS TODAY, ELSEVIER SCIENCE, KIDLINGTON, GB, vol. 7, no. 7-8, July 2004 (2004-07), pages 32-39, XP004599993 ISSN: 1369-7021
- AARON R. MOORE, HAIDOO KWEN, ALICIA M. BEATTY, ERIC A. MAATTA: "Organoimido-polyoxometalates as polymer pendants" CHEMICAL COMMUNICATIONS, 2000, pages 1793-1794, XP002469280 cited in the application
- QI LI, JENNIFER A. LEWIS: "Nanoparticle Inks for Directed Assembly of Three-Dimensional Periodic Structures" ADVANCED MATERIALS, vol. 15, no. 19, 2 October 2003 (2003-10-02), pages 1639-1643, XP002469281

## Description

The present invention relates to internal medical devices having polyelectrolyte-containing extruded regions.

Various medical devices are known which are configured for implantation or insertion into a subject (referred to hereinafter as "internal medical devices").

For example, balloons mounted on the distal ends of catheters are widely used in medical treatment. A balloon may be used, for example, to widen a vessel into which the catheter is inserted or to force open a blocked vessel. The requirements for the strength and size of the balloon vary widely depending on the balloon's intended use and the vessel size into which the catheter is inserted. Some of the most demanding applications for such balloons are in conjunction with balloon angioplasty (e.g., percutaneous transluminal coronary angioplasty or "PCTA") in which catheters are inserted for long distances into extremely small vessels and are used to open stenoses of blood vessels by balloon inflation. These applications require thin-walled, high-strength balloons having predictable inflation properties. Thin walls are necessary, because the balloon's wall thickness limits the minimum diameter of the distal end of the catheter, thereby determining the ease of passage of the catheter through the vascular system and thus treatable vessel size. High strength is necessary because the balloon is used to push open stenoses, and the thin wall of the balloon must not burst under the high internal pressures that are used to accomplish this task (commonly 10 to 25 atmospheres). The balloon elasticity should be relatively low (i.e., the balloon should be substantially non-compliant), so that the diameter is predictable and readily controllable (i.e., small variations in pressure should not cause significant variations in diameter, once the balloon is inflated).

As another example, intraluminal stents or stent grafts are commonly inserted or implanted into body lumens. In some instances, the stent or stent graft is configured to release a therapeutic agent, for example, an anti-thrombogenic agent or an anti-restenosis agent. In one common mode of implantation, the stent is provided in a compact state over an inflatable balloon. This assembly is then advanced to the desired site within a body lumen, whereupon the balloon is inflated and the stent or stent graft is expanded to support the vessel walls. In this process, the stent or stent graft may be subjected to substantial forces and therefore may be required to be mechanically robust.

The above and other challenges are addressed by the present invention. According to one aspect of the present invention, internal medical devices are provided which include one or more extruded regions, each of which may be formed from one or more extruded portions, which extruded portions may, in turn, contain one or more polyelectrolyte species. The one or more extruded regions may be, for example, at least partially freestanding or at least partially disposed over a substrate.

The one or more extruded regions may be formed using various processes, for example, using an extrudable fluid that contains soluble complexes of polycation and polyanion species. In certain embodiments: (a) there may be a non-stoichiometric ratio of ionizable cationic and anionic groups within the polycation and polyanion species of the fluid, (b) the polycation and polyanion species may be of differing molecular weights, and (c) the higher molecular weight species may be provided in a molar excess relative to the lower molecular weight species.

In certain other embodiments, the extruded regions may comprise a reinforcement entity, such as a sol-gel derived reinforcement entity or a nanoparticulate reinforcement entity (e.g., a nanoparticle reinforcement entity comprising derivatized and/or underivatized nanoparticles, such as carbon nanotubes, carbon nanofibers, fullerenes, polyoxometalates, ceramic nanotubes, ceramic nanofibers, phyllosilicates, polyhedral oligomeric silsequioxanes, and combinations thereof).

In certain additional embodiments, polymeric layers are provided over at least a portion of the extruded regions.

In certain further embodiments, the medical devices are provided with a therapeutic agent (e.g., provided within the extruded regions, or on, beneath or within the overlying polymeric layer, if any).

An advantage of the present invention is that, in some embodiments of the invention, medical devices may be provided in which extruded regions are provided with a high degree of spatial accuracy.

Another advantage of the present invention is that, in some embodiments of the invention, medical devices and medical device components may be provided, which are very thin and flexible, have high strength, and/or are substantially non-compliant

These and other aspects, embodiments and advantages of the present invention will become immediately apparent to those of ordinary skill in the art upon reading the disclosure to follow.

Figs. 1A-1B are SEM images of 3-D periodic structures having tetragonal symmetry (filament diameter approx. 1 µm), in accordance with the prior art.

Fig. 2A is a schematic cross-sectional illustration of a balloon catheter in accordance with an embodiment of the present invention.

Fig 2B is a schematic expanded view of the area "b" within Fig. 2A.

Fig. 3 is a simplified schematic diagram of an apparatus for forming medical devices, or portions thereof, in accordance with the invention.

Fig. 4A is a perspective view of an embodiment of a stent, in accordance with the invention.

Fig. 4B is a cross-sectional view of a stent element, taken along line B-B' of Fig. 4A.

Fig. 5 is a side view of an embodiment of an embolic protection filtering device in accordance with the invention.

Fig. 6 is a perspective view of another embodiment of an embolic protection filtering device in accordance with the invention.

According to one aspect of the present invention, internal medical devices (i.e., medical devices adapted for implantation or insertion into a patient) are provided, each of which includes one or more polyelectrolyte-containing extruded regions.

Examples of medical devices for the practice of the present invention include implantable or insertable medical devices and portions thereof, for example, catheters (e.g., renal or vascular catheters), balloons, catheter shafts, guide wires, filters (e.g., vena cava filters), stents (including coronary vascular stents, cerebral, urethral, ureteral, biliary, tracheal, gastrointestinal and esophageal stents), stent delivery systems (e.g., self expanding systems, balloon expandable systems, etc.) stent grafts, cerebral aneurysm filler coils (including Guglilmi detachable coils and metal coils), vascular grafts, myocardial plugs, patches, pacemakers and pacemaker leads, heart valves, vascular valves, biopsy devices, patches for delivery of therapeutic agent to intact skin and broken skin (including wounds); tissue engineering scaffolds for cartilage, bone, skin and other in vivo tissue regeneration, as well as a variety of other devices that are implanted or inserted into the body.

The medical devices of the present invention include medical devices that are used for diagnosis, for systemic treatment, or for the localized treatment of any mammalian tissue or organ. Examples include tumors; organs including the heart, coronary and peripheral vascular system (referred to overall as "the vasculature"), lungs, trachea, esophagus, brain, liver, kidney, bladder, urethra and ureters, eye, intestines, stomach, pancreas, ovary, and prostate; skeletal muscle; smooth muscle; breast; dermal tissue; cartilage; and bone. As used herein, "treatment" refers to the prevention of a disease or condition, the reduction or elimination of symptoms associated with a disease or condition, or the substantial or complete elimination of a disease or condition. Typical subjects are mammalian subjects, more typically human subjects.

As used herein, "extruded regions" are regions that comprise one or more extruded portions, which extruded portions (a) may be solid or hollow (i.e., with one or more lumens), (b) may have any of a variety of lengths, and (c) may have any of a variety of regular and irregular cross-sectional profiles. Examples include ribbon-shaped extruded portions, U-shaped, V-shaped, W-shaped, I-shaped, etc. extruded portions, solid and hollow extruded portions of circular (e.g., rods and tubes), oval, triangular, square, trapezoidal, rhomboidal, pentagonal, star-shaped, etc. cross-section, as well as a near infinite range of additional possible cross-sections.

Where a die or nozzle of fixed cross-section is employed in forming extruded portions in accordance with the present invention, the extruded portions will typically be of near constant cross-section along at least a portion of their lengths. On the other hand, extruded portions may also have sections along their length of variable cross-section, for example, where a die or nozzle is employed whose cross-section is varied during the extrusion of such regions.

Although many of the examples to follow are based on the use of filaments as extruded portions, it should be clear that the present invention is not so limited, and is directed to a wide range of extruded portions of solid and hollow cross-sectional profile, including filaments, among many others.

With this in mind, extruded regions for use in conjunction with the medical devices of the present invention are formed using various direct-write techniques, which involve the formation of filaments from polyelectrolyte-containing fluids, sometimes called "inks." These techniques offer a flexible, inexpensive route for creating complex 3-D structures.

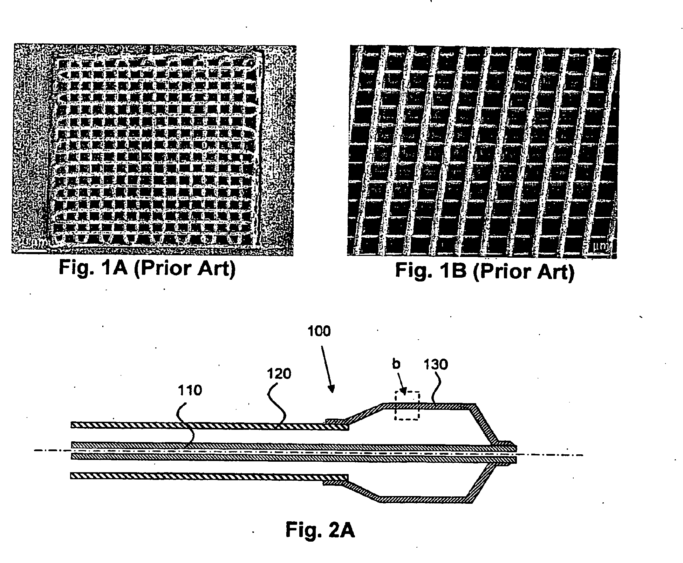

Direct-write assembly of 3-D microperiodic structures using polyelectrolyte inks has been reported using techniques in which polyelectrolyte-containing fluids are routed through nozzles, which can vary widely in size (including, for example, microscale, microcapillary-type deposition nozzles having diameters on the order of 1 micron in diameter, for example, ranging from 0.1 to 0.5 to 1 to 5 to 10 µm in diameter), onto a substrate that is submerged within a deposition reservoir, whereupon the emerging fluid rapidly solidifies to form a solidified structure. An example of such a structure is illustrated in Figs. 1A and 1B, taken from Gratson, G.M.; Lewis, J.A., "Phase Behavior and Rheological Properties of Polyelectrolyte Inks for Direct-Write Assembly," Langmuir 2005, 21, 457-464.

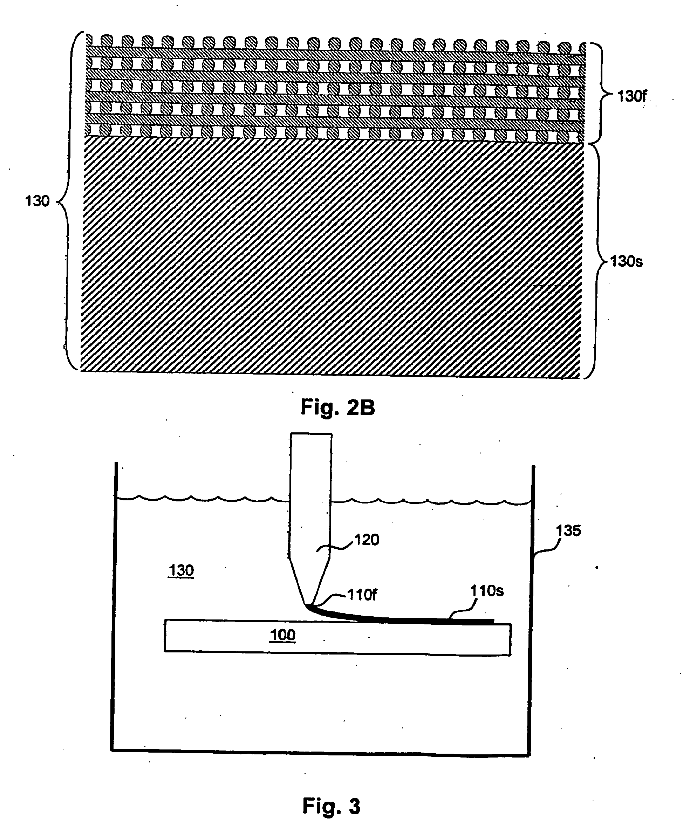

A simplified schematic diagram of an apparatus for carrying out such a procedure is shown in Fig. 3, in which a polyelectrolyte-containing fluid 110f is extruded through a nozzle 120 and onto a substrate 100, which is submerged within a deposition reservoir 135. Upon contacting the fluid 130 in the reservoir 135, the extruded polyelectrolyte-containing fluid 110f solidifies to form a solidified filament 110s on the substrate 100.

Such techniques are readily adaptable to robotic deposition and offer tremendous flexibility for forming a wide variety of structures at very small scales, for example, by direct writing of a continuous ink filament in a layer upon layer fashion. Depending on the spacing between filaments, these structures may be solid or porous. Moreover, these structures may comprise self- supported, spanning filaments, they may have tightly angled features, and so forth.

In addition to the above non-woven structures, when using two or more nozzles having independent movement, woven structures in essentially endless variety may be obtained. For example, an apparatus may be employed where a set of nozzles moves in the X direction ("nozzles X") and a single nozzle moves in the Y direction ("nozzle Y"). In this apparatus, the nozzles X are split into two sets, set X_{A} containing nozzles 1,3,5, etc. and set X_{B} containing nozzles 2,4,6, etc. In a first step, nozzle set X_{A} precede nozzle set X_{B} while nozzle Y is moving down in between the two sets of nozzles X_{A} and X_{B}. In the next step, Nozzles X_{B} precede nozzles X_{A} while nozzle Y is moving backward. Note that this structure is formed in much the same manner as a Persian carpet. There are, of course, many other variations by which filamentous structures may be woven.

In the present invention, deposition times may be reduced by routing the polyelectrolyte-containing fluids through a distributor that contains multiple orifices, thereby depositing multiple extruded portions simultaneously.

Polyelectrolyte-containing fluids for use in the above and other techniques commonly contain high concentrations of soluble polyelectrolyte complexes, which may be formed by combining polyanions and polycations within solution that contains polar solvent species (e.g., water, polar organic species such as lower alcohols, or a combination thereof). Examples of polyanions and polycations for forming soluble polyelectrolyte complexes include polyacrylic acid, polyethylenimine, and polyallylamine hydrochloride, among others. Further polyanions and polycations are described below.

Soluble polyelectrolyte complexes may be formed from polyanions and polycations, for instance, by combining non-stoichiometric mixtures of these species under specific conditions. For example, to create a polyelectrolyte-containing fluid of a desired fluidity, soluble complexes comprised of different molecular weight polyions may be mixed together at a nonstoichiometric ratio of charged groups, with the higher molecular weight species being in excess in the solution, under ionic strength conditions that promote polyelectrolyte exchange reactions, thereby yielding a homogeneous fluid. For example, aqueous solutions containing on the order of about 40-50 wt % soluble polyelectrolyte complexes and having a viscosity on the order of about 5-150 pascal-seconds have been reported.

When deposited within a coagulation reservoir (e.g., one that contains a mixture of alcohol and water, etc.), concentrated polyelectrolyte-containing fluids such as those described above are known to solidify (sometimes referred to as "coagulation") to form self supporting extruded structures, such as those formed from extruded portions such as filaments or rods (see, e.g., Figs. 1A and 1B above.) The reservoir composition has a strong influence on the elasticity of the fluid. As a specific example, the shear elastic modulus of an polyelectrolyte-containing fluid containing a polyacrylic acid/polyethylenimine complex, in which the ratio of anionic groups [-COO] to cationic groups [-NH₃⁺] is about 5.7:1, has been reported to rise dramatically from about 1 Pa prior to deposition within a reservoir containing isopropyl alcohol and water, to about 10⁵ Pa after deposition. Under these conditions, the fluid is able to flow and adhere to the substrate and to any underlying patterned layer(s), while having sufficient elastic modulus after deposition to retain its shape.

Additional information concerning the formation of polyelectrolyte filaments from polyelectrolyte-containing fluids may be found, for example, in Gratson, G.M. and Lewis, J.A., "Phase Behavior and Rheological Properties of Polyelectrolyte Inks for Direct-Write Assembly," Langmuir 2005, 21, 457-464; Gratson et al., "direct writing of three-dimensional webs," Nature 2004, 428, 386; Philipp, B.; Dautzenberg, H.; Linow, K. J.; Kötz, J.; Dawydoff, W. "Polyelectrolyte complexes-recent developments and open problems," Prog. Polym. Sci. 1989, 14, 91-172; Zezin, A. B. and Kabanov, V. A. Russ. Chem. Rev. 1982, 51, 833-855; Zintchenko, A. et al., "Transition Highly Aggregated Complexes-Soluble Complexes via Polyelectrolyte Exchange Reactions: Kinetics, Structural Changes, and Mechanism," Langmuir 2003, 19, 2507-2513.
Further information regarding robotic techniques may be found, for example, in U.S. Patent No. 6,027,326 to Cesarano III et al.; Cesarano III, J., et al., Ceramic Industry (1998) 148, 94; Smay, J. E., et al., Langmuir (2002) 18 (14), 5429; I-Chien Liao, et al., "Controlled release from fibers of polyelectrolyte complexes", Journal of Controlled Release 104 (2005) 347-358.

Many polyelectrolytes are known beyond the polycations and polyanions listed above. As is well known, polyelectrolytes are polymers having charged groups. Usually, the number of these groups in the polyelectrolytes is so large that the polymers are soluble in polar solvents (including water) when they are in ionically dissociated form (also called polyions). Depending on the type of dissociable groups, polyelectrolytes may be classified as polyacids and polybases. When dissociated, polyacids form polyanions, with protons being split off. Polyacids include inorganic, organic and bio-polymers. Examples of polyacids are polyphosphoric acids, polyvinylsulfuric acids, polyvinylsulfonic acids, polyvinylphosphonic acids and polyacrylic acids. Examples of the corresponding salts, which are also called polysalts, are polyphosphates, polyvinylsulfates, polyvinylsulfonates, polyvinylphosphonates and polyacrylates. Polybases contain groups which are capable of accepting protons, e.g., by reaction with acids, with a salt being formed. Examples of polybases having dissociable groups within their backbone and/or side groups are polyallylamine, polyethylimine, polyvinylamine and polyvinylpyridine. By accepting protons, polybases form polycations. Some polyelectrolytes have both anionic and cationic groups, but nonetheless have a net positive charge (in which case they are referred to herein as "polycations") or negative charge (in which case they are referred to herein as "polyanions"), depending on the surrounding pH.

Suitable polyelectrolytes for use in accordance with the invention may be selected from various biopolymers, for example, alginic acid, gummi arabicum, nucleic acids, pectins and proteins, from various chemically modified biopolymers such as carboxymethyl cellulose and lignin sulfonates, and from various synthetic polymers such as polymethacrylic acid, polyvinylsulfonic acid, polyvinylphosphonic acid and polyethylenimine, among many others.

Specific examples from which polycations suitable for the practice of the present invention may be selected include the following: polyamines, including polyamidoamines, poly(amino methacrylates) including poly(dialkylaminoalkyl methacrylate) such as poly(dimethylaminoethyl methacrylate) and poly(diethylaminoethyl methacrylate), polyvinylamines, polyvinylpyridines including quaternary polyvinylpyridines such as poly(N-ethyl-4-vinylpyridine), poly(vinylbenzyltrimethylamines), polyallylamines such as poly(allylamine hydrochloride) (PAH), poly(diallyldialklylamines) such as poly(diallyldimethylammonium chloride), spermine, spermidine, hexadimethrene bromide (polybrene), polyimines including polyalkyleneimines such as polyethyleneimines, polypropyleneimines and ethoxylated polyethyleneimines, polycationic peptides and proteins, including histone polypeptides and polymers containing lysine, arginine, ornithine and combinations thereof including poly-L-lysine, poly-D-lysine, poly-L,D-lysine, poly-L-arginine, poly-D-arginine, poly-D,L-arginine, poly-L-ornithine, poly-D-ornithine, poly-L,D-ornithine, gelatin, albumin, protamine (e.g., protamine sulfate), and polycationic polysaccharides such as cationic starch, chitosan and chitosan derivatives such as chitosan 9Poly-[ß-(1,4)-2-amino-2-desoxy-D-gluco-pyranose], as well as copolymers, derivatives and combinations of the preceding, among various others.

Specific examples from which polyanions suitable for the practice of the present invention may be selected include the following: (a) polysulfonates such as polyvinylsulfonates, poly(styrenesulfonates) such as poly(sodium styrenesulfonate) (PSS), sulfonated poly(tetrafluoroethylene), sulfonated polymers such as those described in U.S. Patent No. 5,840,387, including sulfonated styrene-ethylene/butylene-styrene triblock copolymers, sulfonated styrenic homopolymers and copolymer such as a sulfonated versions of the polystyrene-polyolefin copolymers described in U.S. Patent No. 6,545,097 to Pinchuk et al., which polymers may be sulfonated, for example, using the processes described in U.S. Patent No. 5,840,387 and U.S. Pat. No. 5,468,574, as well as sulfonated versions of various other homopolymers and copolymers; (b) polysulfates such as polyvinylsulfates, sulfated and non-sulfated glycosaminoglycans as well as certain proteoglycans, for example, heparin, heparin sulfate, chondroitin sulfate, keratan sulfate, dermatan sulfate; (c) polycarboxylates such as acrylic acid polymers and salts thereof (e.g., ammonium, potassium, sodium, etc.), for instance, those available from Atofina and Polysciences Inc., methacrylic acid polymers and salts thereof (e.g., EUDRAGIT, a methacrylic acid and ethylacrylate copolymer), carboxymethylcellulose, carboxymethylamylose, and carboxylic acid derivatives of various other polymers, polyanionic peptides and proteins such as glutamic acid polymers and copolymers, aspartic acid polymers and copolymers, polymers and copolymers of uronic acids such as mannuronic acid, galatcuronic acid and guluronic acid, and their salts, for example, alginic acid and sodium alginate polyanions, hyaluronic acid polyanions, gelatin, and carrageenan polyanions; (d) polyphosphates such as phosphoric acid derivatives of various polymers; (e) polyphosphonates such as polyvinylphosphonates; (f) as well as copolymers, derivatives and combinations of the preceding, among various others.

Biodegradable inks may be employed in various embodiments of the invention. For example, such inks may be used to temporarily shield another material, for example, bioerodable metals made from magnesium, iron, magnesium alloys (e.g., those comprising calcium), or iron alloys, among others.

In the case where one provides a device substrate in the form of a rod or a tube (e.g., a guidewire, catheter, stent, etc.), one may extrude ink on the device such that the device is covered with a layer of ink on all sides (e.g., with the ink extruded in the shape of a tube), or only on selected sides (e.g., with the ink extruded in the shape of one or more ribbons). In the specific example of a stent that is provided with open spaces (cells) along the surface of the same, these open spaces may, or may not, be covered depending on the viscosity and speed of the extrusion.

One may extrude multiple polyelectrolyte-containing layers of the same composition or of differing compositions, with the latter being useful to create extruded layers with different therapeutic agents. Multiple layers may be extruded at the same time, or at different times. Extrusion at different times may be useful in some embodiments, for example, where layers containing two therapeutic agents are deposited and it is desired to determine how much of a specific agent has been deposited, for instance, by measuring the weight of the deposited layer.

As seen from the above, extruded regions of use in the present invention are generally deposited upon some type of substrate such that the substrate is wholly or partially covered by the same. Substrates for the practice of the present invention include substrates that are incorporated into the finished medical device, as well as substrates that merely acts as templates for deposition and which are not found in the finished device (although a residue of the substrate may remain). The substrates are commonly formed from ceramic, metallic, polymeric and other high molecular weight materials, including stable and disintegrable materials.

Ceramic substrates may be selected, for example, from substrates containing one or more of the following: metal oxides, including aluminum oxides and transition metal oxides (e.g., oxides of titanium, zirconium, hafnium, tantalum, molybdenum, tungsten, rhenium, and iridium); silicon-based ceramics, such as those containing silicon nitrides, silicon carbides and silicon oxides (sometimes referred to as glass ceramics); calcium phosphate ceramics (e.g., hydroxyapatite); and carbon-based, ceramic-like materials such as carbon nitrides.

Metallic substrates may be selected, for example, from substrates containing one or more of the following: metals (e.g., biostable metals such as gold, platinum, palladium, iridium, osmium, rhodium, titanium, tantalum, tungsten, and ruthenium, and bioerodable metals such as magnesium and iron), metal alloys comprising iron and chromium (e.g., stainless steels, including platinum-enriched radiopaque stainless steel), alloys comprising nickel and titanium (e.g., Nitinol), alloys comprising cobalt and chromium, including alloys that comprise cobalt, chromium and iron (e.g., elgiloy alloys), alloys comprising nickel, cobalt and chromium (e.g., MP 35N) and alloys comprising cobalt, chromium, tungsten and nickel (e.g., L605), alloys comprising nickel and chromium (e.g., inconel alloys), and bioerodable metal alloys, such as alloys of magnesium or iron in combination with Ce, Ca, Zn, Zr and/or Li.

Substrates containing polymers and other high molecular weight materials may be selected, for example, from substrates containing one or more of the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides, polyamidimides, polyesterimides, and polyetherimides; polysulfone polymers and copolymers including polyarylsulfones and polyethersulfones; polyamide polymers and copolymers including nylon 6,6, nylon 12, polyether-block co-polyamide polymers (e.g., Pebax® resins), polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinylacetate copolymers (EVA), polyvinylidene chlorides, polyvinyl ethers such as polyvinyl methyl ethers, vinyl aromatic polymers and copolymers such as
polystyrenes, styrene-maleic anhydride copolymers, vinyl aromatic-hydrocarbon copolymers including styrene-butadiene copolymers, styrene-ethylene-butylene copolymers (e.g., a polystyrene-polyethylene/butylene-polystyrene(SEBS) copolymer, available as Kraton® G series polymers), styrene-isoprene copolymers (e.g., polystyrene-polyisoprene-polystyrene), acrylonitrile-styrene copolymers, acrylonitrile-butadienestyrene copolymers, styrene-butadiene copolymers and styrene-isobutylene copolymers (e.g., polyisobutylene-polystyrene block copolymers such as SIBS), polyvinyl ketones, polyvinylcarbazoles, and polyvinyl esters such as polyvinyl acetates; polybenzimidazoles; ionomers; polyalkyl oxide polymers and copolymers including polyethylene oxides (PEO); polyesters including polyethylene terephthalates, polybutylene terephthalates and aliphatic polyesters such as polymers and copolymers of lactide (which includes lactic acid as well as d-,l- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of polylactic acid and polycaprolactone is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (PEP), modified ethylene-tetrafluoroethylene copolymers (ETFE), and polyvinylidene fluorides (PVDF); silicone polymers and copolymers; polyurethanes; p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosaminoglycans such as hyaluronic acid; as well as blends and further copolymers of the above.

In certain embodiments of the invention, the polyelectrolyte-containing extruded portions are reinforced using one or more reinforcement entities. The reinforcement entities may be least partially inorganic in nature, and they may be provided before, during or after the extruded portions are solidified.

In one example, the extruded portions may be reinforced via one or more sol-gel-derived species. In a typical sol-gel process, precursor materials, typically inorganic metallic and semi-metallic salts, metallic and semi-metallic complexes/chelates, metallic and semi-metallic hydroxides, or organometallic and organo-semi-metallic compounds such as metal alkoxides and alkoxysilanes, are subjected to hydrolysis and condensation (also referred to as polymerization) reactions, thereby forming a "sol". For example, an alkoxide of choice (such as a methoxide, ethoxide, isopropoxide, tert-butoxide, etc.) of a semi-metal or metal of choice (such as silicon, aluminum, zirconium, titanium, tin, hafnium, tantalum, molybdenum, tungsten, rhenium, iridium, etc.) may be dissolved in a suitable solvent, for example, in one or more alcohols. Subsequently, water or another aqueous solution, such as an acidic or basic aqueous solution (which aqueous solution can further contain organic solvent species such as alcohols) is added, causing hydrolysis and condensation to occur.

In the present invention, on the other hand, sol-gel precursors (e.g., alkoxy silanes or metal alkoxides such as those described above), may be added to the alcohol/water reservoir fluids. For example, reservoir fluids may be provided in which the sol-gel precursors are sufficiently stable, such that hydrolysis and condensation processes do not proceed substantially until the polyelectrolyte-containing fluids are introduced to the reservoir fluids. See, for example, D. Wang and F. Caruso, "Polyelectrolyte-Coated Colloid Spheres as Templates for Sol-Gel Reactions," *Chem. Mater.,* 200, *14*, 1909-1903, in which overcoming sensitivity of sol-gel precursors to water by optimizing conditions such that reactions are localized within polyelectrolyte coatings on colloids is discussed in conjunction with the formation of sol-gel-derived hollow spheres.

Without wishing to be bound by theory of operation, it is believed that after the polyelectrolyte-containing fluid contacts the reservoir fluid, these precursors may diffuse at least partially into the deposited polyelectrolyte-containing structures. Hydrolysis and condensation of the precursors may then occur, producing small (e.g., colloidal size) inorganic particles.

In another example, the extruded portions may be reinforced by including particles within the polyelectrolyte-containing fluid.

Examples of suitable particles for this purpose include nanoparticles, which are particles having at least one dimension (e.g., the thickness for a nanoplate or nanoribbon, the diameter for a nanosphere, nanocylinder or nanotube, etc.) that is less than 100 nm. Hence, for example, nanoplates and nanoribbons typically have at least one dimension that is less than 100 nm, nanofibers typically have at least two orthogonal dimensions that are less than 100 nm (e.g., the diameter for cylindrical nanofibers), while other nanoparticles typically have three orthogonal dimensions that are less than 100 nm (e.g., the diameter for nanospheres).

Nanoparticles suitable for use in the polyelectrolyte-containing fluid may be selected, for example, from carbon, ceramic and metallic nanoparticles including nanoplates, nanotubes, and nanospheres, and other regular and irregular nanoparticles. Specific examples of nanoplates include synthetic or natural phyllosilicates including clays and micas (which may optionally be intercalated and/or exfoliated) such as montmorillonite, hectorite, hydrotalcite, vermiculite and laponite. Specific examples of nanotubes and nanofibers include single-wall and multi-wall (including so-called "few-wall") carbon nanotubes, such as fullerene nanotubes, vapor grown carbon fibers, alumina nanofibers, titanium oxide nanofibers, tungsten oxide nanofibers, tantalum oxide nanofibers, zirconium oxide nanofibers, and silicate nanofibers such as aluminum silicate nanofibers. Specific examples of further nanoparticles (e.g., nanoparticles having three orthogonal dimensions that are less than 1000 nm) include fullerenes (e.g., "Buckey ball"), silica nanoparticles, aluminum oxide nanoparticles, titanium oxide nanoparticles, tungsten oxide nanoparticles, tantalum oxide nanoparticles, zirconium oxide nanoparticles, dendrimers, monomeric silicates such as polyhedral oligomeric silsequioxanes (POSS), including various functionalized POSS and polymerized POSS, and polyoxometalates (POMs).

Specific examples of nanoparticles for the practice of the present invention include polyoxometalates (POMs). POMs are a large class of nanosized, anionic, metal and oxygen containing molecules. Polyoxometalates have been synthesized for many years (the first known synthesis dates back to 1826) they readily self assemble under appropriate conditions (e.g., acidic aqueous media), and they are quite stable. POMs comprise one or more types of metal atoms, sometimes referred to as addenda atoms (commonly molybdenum, tungsten, vanadium, niobium, tantalum or a mixture of two or more of these atoms), which with the oxygen atoms form a framework (sometimes referred to as the "shell" or "cage") for the molecule. More specific examples include V^{V}, Nb^{V}, M^{VI} and W^{VI}, among others. Some POMs further comprise one or more types of central atoms, sometimes referred to as heteroatoms, which lie within the shell that is formed by the oxygen and addenda atoms. A very wide variety of elements (i.e., a majority of elements in the periodic table) may act as heteroatoms, with some typical examples being P⁵⁺, As⁵⁺, Si⁴⁺, Ge⁴⁺, B³⁺, and so forth. In certain cases, one or more of the oxygen atoms within the POM is/are substituted by S, F, Br and/or other p-block elements. Materials for forming POMs may be obtained, for example, from Sigma Aldrich and Goodfellow Corp., among other sources.

In certain embodiments, the POMs may have a general formula of A[VₖMoₘWₙNbₒTaₚM_{q}XᵣOₛ]^{y-}. A is at least one counterion, which can include, for example, alkali metal cations, alkaline earth metal cations, ammonium cations, quaternary ammonium cations, d-block cations, f-block cations, various organic or polymeric cations, such as organic and polymeric amines, and combinations thereof, among others. V, Mo, W, Nb, Ta and M are addenda atoms, where V is vanadium, Mo is molybdenum, W is tungsten, Nb is niobium, Ta is tantalum, and M is at least one f- or d-block element having at least one d-electron, other than vanadium, molybdenum, tungsten, niobium, or tantalum. X is at least one heteroatom selected from p-, d-, and f-block elements, other than oxygen. In addition, k can range from 0 to 30, m can range from 0 to 160, n can range from 0 to 160, o can range from 0 to 30, p can range from 0 to 10, q can range from 0 to 30, r can range from 0 to 30, s is sufficiently large such that y is greater than zero, and the sum of k, m, n, o, and p is greater than or equal to four. In certain embodiments, one or more of the oxygen atoms within the POM is/are substituted by S, F, Br and/or other p-block elements.

Derivatized POMs are also being developed constantly in which organic compounds, including polymers and non-polymers, are covalently linked or otherwise associated with POMs. Examples include POM derivatives where one or more organic compounds are bonded directly to the POM framework (e.g., to addenda atoms) and/or bonded to POM heteroatoms. For instance, POM derivatives may be prepared by a variety of techniques including techniques where organic compounds are covalently bound to POM addenda atoms or heteroatoms by imido linkages. For further information, see, e.g., Peng, Z., "Rational synthesis of covalently bonded organic-inorganic hybrids," Angew Chem Int Ed Engl. 2004 Feb 13; 43(8), 930-5; Moore, A.R et al., "Organoimido-polyoxometalates as polymer pendants," Chem. Commun. 2000, 1793-1794; Hu Changwen et al., "Polyoxometalate-based organic-inorganic hybrid materials," C.J.I. 2001 June 1, 3(6), 22.

Further examples of derivatized and non-derivatized POMs that are useful for the present invention may be selected from those set forth in U.S. Patent No. 2004/0230086 to Okum et al.; U.S. Patent No. 2003/0157012 to Pope et al., Pope, M. T. in Heteropoly and Isopoly Oxometalates, Springer Verlag, 1983, and Chemical Reviews, vol. 98, no. 1, pp: 1-389, 1998.

Because many POMs and their derivatives are soluble, they may be readily introduced into the polyelectrolyte-containing fluids prior to forming extruded regions for use in the present invention.

Further specific examples of nanoparticles for the practice of the present invention include derivatized and non-derivatized carbon nanotubes and carbon nanofibers having a diameter ranging from 0.5 nm to 200 nm. In this regard, carbon nanotubes, especially single-wall carbon nanotubes (SWNT), have remarkable electrical and mechanical properties, and show great promise for enhancing strength in composites, such as polymer composites.

In order to maximize their properties, it is typically desirable to use disperse the carbon nanotubes. For example, is known that various nanoparticles, including carbon nanotubes, may be partially oxidized by refluxing in strong acid (e.g., nitric acid) to form carboxylic acid groups (which ionize to become negatively charged carboxyl groups) on the nanoparticles thereby forming derivatized nanoparticles. Consequently, relatively stable and uniform suspensions of the nanoparticles may be achieved, due at least in part to electrostatic stabilization effects.

Nanoparticles, including carbon nanotubes, may also be dispersed in polar fluids, including aqueous fluids, by introducing various dispersing species, which, without wishing to be bound by theory, are believed to wrap, encapsulate or otherwise coat the nanoparticles, thereby providing hybrid structures which may, for example, render the nanotubes dispersible in water and in other compatible solvents, among other properties. Examples of such dispersing species include various dispersing polymers, such as polyvinyl pyrrolidone, polystyrene sulfonate, poly(1-vinyl pyrrolidone-co-vinyl acetate), poly(1-vinyl pyrrolidone-co-acrylic acid), poly(1-vinyl pyrrolidone-co-dimethylaminoethyl methacrylate), polyvinyl sulfate, poly(sodium styrene sulfonic acid-co-maleic acid), polyethylene oxide, polypropylene oxide, dextran, dextran sulfate, bovine serum albumin, poly(methyl methacrylate-co-ethyl acrylate), polyvinyl alcohol, polyethylene glycol, polyallyl amine, as well as copolymers and combinations thereof.

In other embodiments, non-derivatized or derivatized nanoparticles, including derivatized single-wall carbon nanotubes, among others, may be dispersed in an aqueous system wherein the particles are surrounded by surfactant molecules. (Note that non-derivatized carbon nanotubes are generally amphiphobic in that they are difficult to solubilize in both polar and non-polar solvents; thus, one generally creates functional groups on their surfaces, prior to dissolving them, even with the aid of surfactants.) "Surfactants" are generally molecules having polar and non-polar ends and which are able to position themselves at interfaces to lower the surface tension between immiscible chemical species. Without wishing to be bound by theory, in certain embodiments, the non-polar end of the surfactant molecule is believed to interact with the nanoparticle (e.g., nanotube), while the polar end is believed to interact with aqueous or other polar media, for example, in a micelle-type arrangement. Nonionic, anionic, and cationic surfactants, may be used in an appropriate solvent medium, such as water and/or a polar organic species.

Examples of nonionic surfactants from which a suitable nonionic surfactant may be selected include TRITON-X surfactants (from Union Carbide; examples of TRITON-X surfactants include, but are not limited to, alkylaryl polyethether alcohols, ethoxylated propoxylated C₈-C₁₀ alcohols, t-octylphenoxypolyethoxyethanbl, polyethylene glycol tert-octylphenyl ether, and polyoxyethylene isooctylcyclohexyl ether), SARKOSYL L surfactants (also known as N-lauroylsarcosine or N-dodecanoyl-N-methylglycine), BRIM surfactants (ICI Americas, Inc.; examples of BRIJ surfactants are polyethylene glycol dodecyl ether, polyethylene glycol lauryl ether, polyethylene glycol hexadecyl ether, polyethylene glycol stearyl ether, and polyethylene glycol oleyl tther), PLUROMC surfactants (BASF Corporation; PLURONIC surfactants are block copolymers of polyethylene and polypropylene glycol), TWEEN surfactants (ICI Americas, Inc; TWEEN surfactants include polyethylene glycol sorbitan monolaurate, also known as polyoxyethylenesorbitan monolaurate, polyoxyethylene monostearate, polyoxyethylenesorbitan tristearate, polyoxyethylenesorbitan monooleate, polyoxyethylenesorbitan trioleate, and polyoxyethylenesorbitan monopalmitate), and combinations thereof. Alkylaryl polyethether alcohols, commercially known as TRITON-X surfactants, are commonly used as non-ionic surfactants for dispersing nanoparticles including nanotubes.

Examples of anionic surfactants from which a suitable anionic surfactant may be selected include, for example, sodium dodecyl sulfate (SDS), sodium dodecyl sulfonate (SDSA), sodium alkyl allyl sulfosuccinate (TREM), SARKOSYL NL surfactants (Ciba-Geigy UK, Limited; other nomenclature for SARKOSYL NL surfactants include N-lauroylsarcosine sodium salt, N-dodecanoyl-N-methylglycine sodium salt and sodium N-dodecanoyl-N-methylglycinate), and combinations thereof. A commonly used anionic surfactant is sodium dodecyl sulfate (SDS).

Examples of cationic surfactants from which a suitable cationic surfactant may be selected include, for example, chitosan and its derivatives, dodecyltrimethylammonium bromide (DTAB), cetyltrimethylammonium bromide (CTAB), cetyltrimethylammonium chloride (CTAC) and combinations thereof.

To facilitate the preparation and dispersion of nanoparticles (e.g., single-wall carbon nanotubes), a mixture including water (and/or another polar solvent), the nanoparticles, and a dispersing species or surfactant, may be subjected to high-shear mixing. To further facilitate dispersion, the mixture may be subjected to sonication or ultrasonication. After forming a dispersion of the nanoparticles (e.g., nanotubes), individually-dispersed particles may be separated from those particles that are dispersed in aggregates (e.g., nanotube bundles or ropes) as well as from other non-nanoparticle solids. Centrifugation and ultracentrifugation may be suitable means for separating the individually-dispersed nanoparticles from the aggregates and other solids. Taking carbon nanotubes as an example, with centrifugation, the nanotube aggregates and other non-nanotube solids tend to concentrate in the sediment at the bottom of the centrifuge tube, while the individually-dispersed nanotubes remain suspended in the supernatant.

Further information on forming carbon nanotube dispersions using dispersing species or surfactants such as those above may be found, for example, in U.S. Patent Application Pub. No. 2004/0040834 to Smalley et al., O'Connell, et al., "Reversible Water Solubilization of Single-Walled Carbon Nanotubes by Polymer Wrapping," Chem. Phys. Lett. 2001, 324, 265-271, and "Polymer-Wrapped Single-Wall Carbon Nanotubes," Int. Pat. Publ. No. WO 02/016257, filed Aug. 23, 2001.

In other embodiments, derivatized nanoparticles, including derivatized carbon nanotubes, may be dispersed by linking them, for example, to poly(propionylethylenimine-co-ethylenimine), to poly(ethylene glycol) or to various other polymeric and non-polymeric species. See, e.g., J.E. Riggs et al, "Optical Limiting Properties of Suspended and Solubilized Carbon Nanotubes," J. Phys. Chem. B 2000, 104, 7071-7076 and E. Menna, et al., "shortened single-walled nanotubes functionalized with poly(ethylene glycol): preparation and properties", Arkivoc 2003 Part 12, 64-73. In the specific instance of carbon nanotubes, functional groups for covalent linking may be formed by treating the nanotubes with an oxidizing acid. For example, in the preceding papers, carbon nanotubes treated in an acid oxidative cutting and etching process are exposed to SOCl₂ (thionyl chloride), followed by amidation with poly(propionylethylenimine-co-ethylenimine) or poly(ethylene glycol) monoamine.

As with the POMs above, once dispersed, the nanoparticles, including carbon nanotubes, are able to be introduced to polyelectrolyte-containing fluids, which may subsequently be used to form extruded portions, such as filaments, among others.

As noted above, in accordance with some embodiments of the present invention, extruded regions are formed upon underlying substrates that become incorporated into the finished medical devices. As one specific example, one or more extruded regions in accordance with the present invention may be built upon a preexisting balloon, such as a Pebax® balloon.

In some embodiments, on the other hand, the underlying substrate merely acts as a template (e.g., as a mold) for application of the extruded region, and the extruded region is freed from the substrate after forming the same (e.g., by releasing it from the template or destroying all or a portion of the template). The extruded region is applied in some instance to the inside of the removable substrate, and is applied in other instances to the outside of the removable substrate. The resulting free-standing material may be, for example, used as is, applied to another substrate, sandwiched between other layers, and so forth.

Where the extruded region is provided over a substrate, it can extend over all or only a portion of the substrate. For example, extruded regions may be provided over multiple surface portions of an underlying substrate, and may be provided in any shape or pattern.

In certain embodiments, a polymeric layer is provided over the extruded region(s), thereby covering the same. Such a polymeric layer may be provided, for example, to contain any debris in the unlikely event that the extruded region becomes damaged (e.g., in the unlikely event of a balloon burst), or a therapeutic agent may be associated with such an outer polymer layer, for example, to provide for in vivo delivery of the same. Such polymeric layers can be formed from one or more polymers selected from the polymers described above for use in forming polymer substrates, using, for example, thermoplastic or solvent processing techniques.

As indicated above, in some embodiments of the invention, one or more therapeutic agents may be associated with the medical devices of the invention, for example, by incorporating them into or onto the extruded region, or into, onto, or beneath the optional polymeric layer. Among other effects, this may give the internal medical devices of the present invention a therapeutic-agent-releasing function upon implantation or insertion. For instance, therapeutic agents may be included within the fluid that is used to form one or more extruded portions forming the extruded region, applied onto the extruded region after its formation, included within a melt or solution that is used to form the optional polymeric layer, applied onto the optional polymeric layer after it is formed, and so forth.

"Therapeutic agents," "drugs," "bioactive agents" "pharmaceuticals," "pharmaceutically active agents", and other related terms may be used interchangeably herein and include genetic and non-genetic therapeutic agents. Therapeutic agents may be used singly or in combination.

A wide range of therapeutic agent loadings can be used in conjunction with the devices of the present invention, with the pharmaceutically effective amount being readily determined by those of ordinary skill in the art and ultimately depending, for example, upon the condition to be treated, the nature of the therapeutic agent itself, the tissue into which the dosage form is introduced, and so forth.

Therapeutic agents may be selected, for example, from the following: adrenergic agents, adrenocortical steroids, adrenocortical suppressants, alcohol deterrents, aldosterone antagonists, amino acids and proteins, ammonia detoxicants, anabolic agents, analeptic agents, analgesic agents, androgenic agents, anesthetic agents, anorectic compounds, anorexic agents, antagonists, anterior pituitary activators and suppressants, anthelmintic agents, anti-adrenergic agents, anti-allergic agents, anti-amebic agents, anti-androgen agents, anti-anemic agents, anti-anginal agents, anti-anxiety agents, antiarthritic agents, anti-asthmatic agents, anti-atherosclerotic agents, antibacterial agents, anticholelithic agents, anticholelithogenic agents, anticholinergic agents, anticoagulants, anticoccidal agents, ariticonvulsants, antidepressants, antidiabetic agents, antidiuretics, antidotes, antidyskinetics agents, anti-emetic agents, anti-epileptic agents, anti-estrogen agents, antifibrinolytic agents, antifungal agents, antiglaucoma agents, antihemophilic agents, antihemophilic Factor, antihemorrhagic agents, antihistaminic agents, antihyperlipidemic agents, antihyperlipoproteinemic agents, antihypertensives, antihypotensives; anti-infective agents, anti-inflammatory agents, antikeratinizing agents, antimicrobial agents, antimigraine agents, antimitotic agents, antimycotic agents, antineoplastic agents, anti-cancer supplementary potentiating agents, antineutropenic agents, antiobsessional agents, antiparasitic agents, antiparkinsonian drugs, antipneumocystic agents, antiproliferative agents, antiprostatic hypertrophy drugs, antiprotozoal agents, antipruritics, antipsoriatic agents, antipsychotics, antirheumatic agents, antischistosomal agents, antiseborrheic agents, antispasmodic agents, antithrombotic agents, antitussive agents, anti-ulcerative agents, anti-urolithic agents, antiviral agents, benign prostatic hyperplasia therapy agents, blood glucose regulators, bone resorption inhibitors, bronchodilators, carbonic anhydrase inhibitors, cardiac depressants, cardioprotectants, cardiotonic agents, cardiovascular agents, choleretic agents, cholinergic agents, cholinergic agonists, cholinesterase deactivators, coocidiostat agents, cognition adjuvants and cognition enhancers, depressants, diagnostic aids, diuretics, dopaminergic agents, ectoparasiticides, emetic agents, enzyme inhibitors, estrogens, fibrinolytic agents, free oxygen radical scavengers, gastrointestinal motility agents, glucocorticoids, gonad-stimulating principles, hemostatic agents, histamine H2 receptor antagonists, hormones, hypocholestemlemic agents, hypoglycemic agents, hypolipidemic agents, hypotensive agents, HMGCoA reductase inhibitors, immunizing agents, immunomodulators, immunoregulators, immune response modifiers, immunostimulants, immunosuppressants, impotence therapy adjuncts, keratolytic agents, LHRH agonists, luteolysin agents, mucolytics, mucosal protective agents, mydriatic agents, nasal decongestants, neuroleptic agents, neuromuscular blocking agents, neuroprotective agents, NMDA antagonists, non-hormonal sterol derivatives, oxytocic agents, plasminogen activators, platelet activating factor antagonists, platelet aggregation inhibitors, post-stroke and post-head trauma treatment, progestins, prostaglandins, prostate growth inhibitors, prothyrotropin agents, psychotropic agents, radioactive agents, repartitioning agents, scabicides, sclerosing agents, sedatives, sedative-hypnotic agents, selective adenosine A1 antagonists, semtonin antagonisrts, serotonin inhibitors, serotonin receptor antagonists, steroids, stimulants, thyroid hormones, thyroid inhibitors, thyromimetic agents, tranquilizers, unstable angina agents, uricosuric agents, vasoconstrictors, vasodilators, vulnerary agents, wound healing agents, xanthine oxidase inhibitors, and the like.

Numerous additional therapeutic agents useful for the practice of the present invention may be selected from those described in paragraphs [0040] to [0046] of commonly assigned U.S. Patent Application Pub. No. 2003/0236514.

Some specific beneficial agents include paclitaxel, sirolimus, everolimus, tacrolimus, Epo D, dexamethasone, estradiol, halofuginone, cilostazole, geldanamycin, ABT-578 (Abbott Laboratories), trapidil, liprostin, Actinomcin D, Resten-NG, Ap-17, abciximab, clopidogrel, Ridogrel, beta-blockers, bARKct inhibitors, phospholamban inhibitors, and Serca 2 gene/protein, resiquimod, imiquimod (as well as other imidazoquinoline immune response modifiers), human apolioproteins (e.g., AI, AII, AIII, AIV, AV, etc.), vascular endothelial growth factors (e.g., VEGF-2), as well a derivatives of the forgoing, among many others.

Certain specific embodiments of the invention will now be described with reference to the Figures.

Referring now to Fig. 2A, a balloon catheter is shown, which includes an inner guidewire lumen 110, an outer inflation lumen 120, and a balloon 130. Guidewire and inflation lumens are well known in the art and are commonly formed from materials including polyamide polymers and copolymers, such as nylon 12 and polyether-block co-polyamide polymers (e.g., Pebax®), polyesters including polyalkylene terephthalate polymers and copolymers (e.g., thermoplastic polyester elastomers such as Hytrel®, which is a block copolymer containing a hard polybutylene terephthalate segment and soft amorphous segments based on long-chain polyether glycols), polyethylenes (particularly high density polyethylenes), and polyurethanes. Guidewire lumens are commonly provided with lubricious materials on their inner surfaces, for example, polytetrafluoroethylene or high density polyethylene.

Further details regarding the construction of the balloon 130 can be seen from Fig. 2B, which an expanded schematic illustration of area "b" in Fig. 2A. As seen in Fig. 2B, the wall of the balloon 130 includes an extruded region 130f disposed over a substrate region 130s, which in this particular example corresponds to a polymeric, inflatable substrate. A few examples of polymeric materials that may be used as the substrate region 130s for the balloon 130, include polyamide polymers and copolymers, such as nylon 12 and polyether-block co-polyamide polymers (e.g., Pebax®), and polyesters, including polyalkylene terephthalate polymers and copolymers (e.g., polyethylene terephthalate), among many others. Polymeric materials such as the preceding may also be blended, or may be provided in a composite or multi-layer substrate construction, if desired. Typical balloon wall thicknesses range from 10 to 30 micrometers.

In the embodiment illustrated, the extruded region 130f is formed from one or more polyelectrolyte-containing extruded portions (i.e., filaments), which may or may not be reinforced and which may or may not contain a therapeutic agent, as described in more detail above. The filament(s) making up region 130f have typical diameters ranging from 0.1 to 50 micrometers. Specific examples of polyelectrolyte combinations include polyacrylic acid as a polyanion and polyethylenimine or polyallylamine hydrochloride as a polycation, among many others (e.g., selected from those above).

The extruded region 130f shown consists of multiple layers, each of which contains substantially parallel filament segments which may be formed from multiple filaments or a single filament (e.g., by arranging a single filament over the balloon in a manner analogous to that shown in Fig. 1A above, by wrapping a single filament around the balloon in an advancing coil/helix, and so forth). The substantially parallel filament segments within each layer may be oriented at any desired angle with respect to the substantially parallel filament segments of the immediately underlying and/or overlying layer. In Figs. 1A and 1B this angle is approximately 90°. Layers composed of substantially parallel filaments, in which the layer are stacked such that the filaments between layers are at a 90° angle with respect to one another, have been shown to provide very high strength. For instance, Spectra Shield®, a well-known bullet proof composite, contains two unidirectional layers of Spectra® fiber (i.e., a high strength, ultra-high molecular weight polyethylene fiber from Allied Signal), arranged at a 90° angle with respect to each other.

Of course, a near infinite range of woven and non-woven filament orientations may be provided using the direct-write technique described previously or using another suitable deposition method. For example, the filament(s) may be oriented in a fashion like that of the filaments within a spider web, among many other arrangements.

When applying the extruded region 130f, the substrate 130s may be movably mounted, the "ink" distributor(s) may be movably mounted, or both. For example, the substrate 130s may be mounted on a rotating shaft, while an application nozzle may be mounted on a micro-positioning device which moves along 2 orthogonal axes (e.g., along the axis of the rotating shaft and radially inward and outward with respect to the axis of the shaft).

As another example, a three-axis micro-positioning device may be used to deposit the filaments on a stationary substrate. For example, after producing a first layer pattern in an x-y plane, the nozzle may be raised along the z-axis to create a subsequent layer. This process may be repeated until a complete structure is fabricated.

Once the extruded region is completed, an optional polymeric layer (not illustrated), which may or may not contain a therapeutic agent, may be provided over all or a portion of the balloon.

Balloons made by procedures such as those discussed herein may be designed to be flexible, strong and non-compliant.

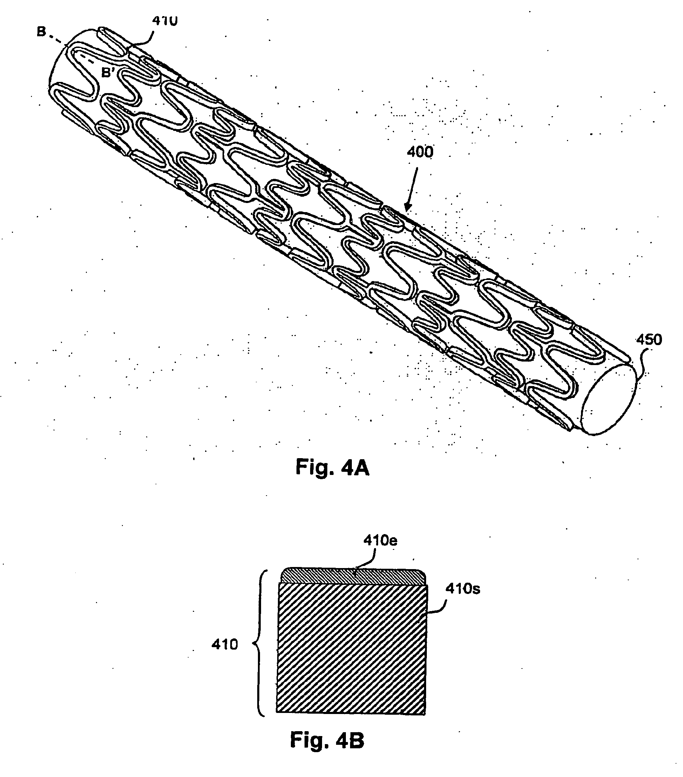

Of course, the present invention has applicability to a wide range of medical devices other than balloon catheters, as noted above. As one specific example, a stent 400, analogous in structure to that illustrated in U.S. Application Publication No. 2005/0182480, is shown in Fig 4A, disposed on a support 450. The stent comprises various interconnected stent elements 410, which form numerous open cells. In contrast to the stent in U.S. Application Publication No. 2005/0182480, and as can be seen in the cross-section schematically illustrated in Fig. 4B, each stent element 410 in Fig. 4A (or only a portion of the stent elements) may comprise an extruded region 410e, disposed over a structural element 410s, which may be formed, for example, from a metal or metal alloy, such as those described above. The extruded region 410e shown is in the shape of a single ribbon, which may be extruded, for example, from a nozzle with a slot shaped orifice, although extruded regions of other shapes or in other numbers (e.g., or multiple filaments, etc.) may clearly be applied. In the embodiment illustrated, the extruded region 410e may comprise an optional therapeutic agent (e.g., an anti-restenotic agent), whereby the agent is locally delivered to the surrounding bodily tissue (e.g., a blood vessel), upon expansion in vivo.

In other specific examples, filters may be created, which employ extruded regions in accordance with the present invention as a filter material. For example, a woven or non-woven extruded region (see the non-woven extruded region of Fig. 1A, among many other possibilities) may be employed as a filter material. The open spaces in the filter material are sized to allow blood flow through the filter material but restrict flow of debris or emboli floating in the body lumen or cavity. For instance, by making the open spaces between extruded filaments on the order about 10 micrometers, red blood cells will be allowed to cross the filter and support, whereas substantially larger species will not. Depending on the tolerance for particulate matter, the open spaces may be, for example, 10 to 100 micrometers or larger.

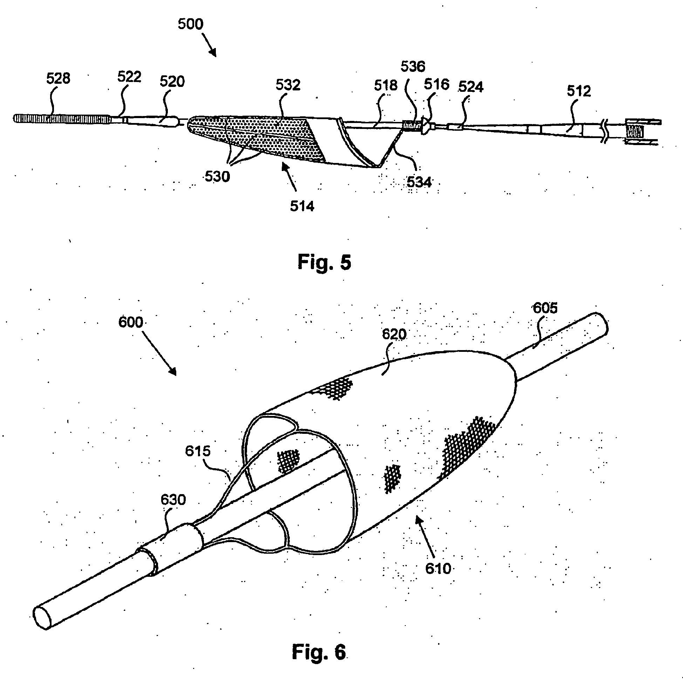

As a specific example, Fig. 5 is a side view of an embolic protection assembly 500, which may be used to filter out embolic debris. Embolic protection assembly 500 includes an elongate shaft 512 having a filter 514 coupled thereto. A proximal stop 516 is adapted and configured to stop a medical device such as a therapeutic catheter from being advanced over the shaft 512 beyond stop 516. Stop 516, thus, can prevent the medical device from being advanced distally over the filter 514. In some embodiments, filter 514 is coupled to a tube 518 slidably disposed over shaft 512. Tube 518 is adapted and configured to allow filter 514 to be advanced over shaft 512 to a desired location. Tube 518 may be held in position by a first stop 520 (e.g., located near the distal end 522 of shaft 512), and a second stop 524 (e.g., generally located proximally of first stop 520). In some embodiments, stop 516 may be attached to tube 518. Shaft 512 may also include a distal tip 528. Distal tip 528 may comprise a "spring tip" or "floppy tip" similar to analogous tips known in the art

Filter 514 may include a filter frame 530, a filter material 532 disposed over the frame 530, and one or more struts 534. In general, filter 514 operates between a first generally collapsed configuration and a second generally expanded configuration for collecting debris in a body lumen. Frame 530 may be comprised of a "self-expanding" shape-memory material such as nickel-titanium alloy to bias filter 514 to be in the second expanded configuration. Strut 534 may be coupled to tube 518 (or shaft 512) by a coupling 536. Coupling 536 may be one or more windings of strut 534 about tube 518 (or shaft 512) or may be a fitting disposed over an end of strut 534 to attach it to tube 518. The assembly 500 shown in Fig. 5 is analogous to that described in U.S. Patent Application Publication No. 2004/0127933, except that the filter material 532 in the filter 514 is an extruded region in accordance with the present invention (e.g., a woven structure, or a non-woven structure like that of Fig. 1A). The extruded region may be, for example, formed over the filter frame 530 or may be preformed (e.g., utilizing a removable support of suitable shape) and attached to the filter frame 530.

Another specific example of an embolic protection assembly 600 is illustrated in Fig. 6, which shows an elongate shaft 605 having a filter 610 coupled thereto. Filter 610 may include a filter material 620 which is attached to one end of a filter frame 615. The opposite end of the filter frame 615 is in the form of two struts, which may be coupled to the elongate shaft 605 by a coupling 630, or by other mechanisms, such as by mechanical bond such as a crimp, by adhesives, by thermal bond such as a weld, and the like. The coupling 630 illustrated is disposed over an end of the filter frame 615 to attach it to shaft 605. In general, the filter 610 may be adapted to operate between a first generally collapsed configuration and a second generally expanded configuration for collecting debris in a body lumen by means of the filter material 620. Frame 615 may be comprised, for example, of a self-expanding shape-memory material such as nickel-titanium alloy for this purpose. The assembly 600 shown in Fig. 6 is analogous to that described in U.S. Patent Application Publication No. 2004/0147955,
except that the filter material 620 in the filter 610 is an extruded region in accordance with the present invention (e.g., a woven structure, or a non-woven structure like that of Fig. 1A).

Although various embodiments of the invention are specifically illustrated and described herein, it will be appreciated that modifications and variations of the present invention are covered by the above teachings without departing from the intended scope of the invention.

## Claims

1. An internal medical device the internal medical device being selected from a graft, a stent, a filter and an inflatable balloon, comprising an extruded region that comprises an extruded portion, said extruded portion comprising a polyelectrolyte, at least a portion of said extruded region is disposed over a permanent underlying or overlying substrate of said internal medical device and said internal medical device being adapted for implantation or insertion into a subject; wherein the extruded region is formed using a direct-write technique, which involves the formation of filaments from polyelectrolyte-containing fluids.

2. The internal medical device of claim 1,
comprising a plurality of extruded regions, or
wherein said extruded region comprises a plurality of said extruded portions, wherein said extruded portions are preferably selected from solid extruded portions, hollow extruded portions and combinations of the same, or
wherein said extruded portion comprises a plurality of polyelectrolytes, or
wherein a polymeric layer is provided over at least a portion of said extruded region.

3. The internal medical device of claim 1, wherein said extruded portion comprises a polycation species and a polyanion species.

4. The internal medical device of claim 3,
wherein said polycation and polyanion species comprise a nonstoichiometric ratio of ionizable cationic and anionic groups, wherein, optionally, said polycation and polyanion species are of differing molecular weight and wherein the polycation and polyanion species of higher molecular weight is provided in a molar excess relative to the polycation and polyanion species of lower molecular weight, or
wherein said polyanion species is polyacrylic acid and wherein said polycation species is selected from polyethylenimine and polyallylamine hydrochloride.

5. The internal medical device of claim 1, wherein said extruded portion comprises a reinforcement entity.

6. The internal medical device of claim 5, wherein said reinforcement entity is a sol-gel derived reinforcement entity.

7. The internal medical device of claim 5, wherein said reinforcement entity is a nanoparticulate reinforcement entity.

8. The internal medical device of claim 7,
wherein said nanoparticulate reinforcement entity comprises nanoparticles selected from derivatized and underivatized carbon nanotubes,
derivatized and underivatized polyoxometalates, polyoxometalates of the formula
A [VₖMoₘWₙNbₒTaₚM_{q}XᵣOₛ]^{y-},
wherein A represents at least one counterion, wherein V, Mo, W, Nb, Ta and M are addenda atoms, wherein M represents at least one f- or d-block element having at least one d-electron, other than vanadium, molybdenum, tungsten, niobium, or tantalum, wherein X is at least one heteroatom selected from p-, d-, and f-block elements other than oxygen, wherein k ranges from 0 to 30, wherein m ranges from 0 to 160, wherein n ranges from 0 to 160, wherein o ranges from 0 to 30, wherein p ranges from 0 to 10, wherein q ranges from 0 to 30, wherein r ranges from 0 to 30, wherein s is sufficiently large such that y is greater than zero, wherein the sum of k, m, n, o, and p is greater than or equal to four, and wherein one or more of the oxygen atoms within the polyoxometalates may optionally be substituted by one or more p-block elements,
derivatized and underivatized carbon nanofibers, derivatized and underivatized fullerenes, derivatized and underivatized ceramic nanotubes, derivatized and underivatized ceramic nanofibers, derivatized and underivatized phyllosilicates, derivatized and underivatized polyhedral oligomeric silsequioxanes, and combinations thereof.

9. The internal medical device of claim 1, wherein a therapeutic agent is provided on or within said extruded portion, wherein a therapeutic agent is optionally provided on, beneath or within said polymeric layer.

10. The medical device of claim 1, wherein at least a portion of said extruded region is freestanding.

11. The medical device of claim 1,
wherein said permanent underlying or overlying substrate is a ceramic, metallic, or polymeric structure, or optionally comprising an inflatable balloon substrate underlying said extruded region.

12. The medical device of claim 1, wherein said extruded portion is in the form of a layer that covers all or a portion of an underlying medical device substrate.

13. The medical device of claim 7, wherein said nanoparticle reinforcement entity comprises nanoparticles ranging from 0,5 to 100 nm in smallest dimension.

## Patentansprüche

1. Inneres Medizinprodukt, wobei das innere Medizinprodukt aus einem Transplantat, einem Stent, einem Filter und einem aufblasbaren Ballon ausgewählt ist, mit einem extrudierten Bereich, der einen extrudierten Abschnitt aufweist, wobei der extrudierte Abschnitt einen Polyelektrolyt aufweist, mindestens ein Abschnitt des extrudierten Bereichs über einem permanenten darunter oder darüber liegenden Substrat des inneren Medizinprodukts angeordnet ist und das innere Medizinprodukt zur Implantation oder Einführung in ein Subjekt geeignet ist; wobei der extrudierte Bereich mit Hilfe einer Direktschreibtechnik gebildet ist, die die Bildung von Filamenten aus polyelektrolythaltigen Fluiden umfaßst.

2. Inneres Medizinprodukt nach Anspruch 1
mit mehreren extrudierten Bereichen, oder
wobei der extrudierte Bereich mehrere der extrudierten Abschnitte aufweist, wobei die extrudierten Abschnitte vorzugsweise aus massiven extrudierten Abschnitten, hohlen extrudierten Abschnitten und Kombinationen daraus ausgewählt sind oder
wobei der extrudierte Abschnitt mehrere Polyelektrolyte aufweist oder
wobei eine Polymerschicht über mindestens einem Abschnitt des extrudierten Bereichs vorgesehen ist.

3. Inneres Medizinprodukt nach Anspruch 1, wobei der extrudierte Abschnitt eine Polykationenart und eine Polyanionenart aufweist.

4. Inneres Medizinprodukt nach Anspruch 3,
wobei die Polykationen- und Polyanionenart ein nicht stöchiometrisches Verhältnis ionisierbarer kationischer und anionischer Gruppen aufweist, wobei optional die Polykationen- und Polyanionenart ein unterschiedliches Molekulargewicht haben und wobei die Polykationen- und Polyanionenart mit höherem Molekulargewicht in einem Molüberschuss relativ zur Polykationen- und Polyanionenart mit niedrigerem Molekulargewicht vorgesehen ist oder wobei die Polyanionenart Polyacrylsäure ist und wobei die Polykationenart aus Polyethylenimin und Polyallylaminhydrochlorid ausgewählt ist.

5. Inneres Medizinprodukt nach Anspruch 1, wobei der extrudierte Abschnitt ein Verstärkungsgebilde aufweist.

6. Inneres Medizinprodukt nach Anspruch 5, wobei das Verstärkungsgebilde ein Sol-Gel-abgeleitetes Verstärkungsgebilde ist.

7. Inneres Medizinprodukt nach Anspruch 5, wobei das Verstärkungsgebilde ein Nanopartikel-Verstärkungsgebilde ist.

8. Inneres Medizinprodukt nach Anspruch 7,
wobei das Nanopartikel-Verstärkungsgebilde Nanopartikel aufweist, die ausgewählt sind aus derivatisierten und nicht derivatisierten Kohlenstoffnanoröhrchen, derivatisierten und nicht derivatisierten Polyoxometallaten,
Polyoxometallaten mit der Formel
A[VₖMoₘWₙNbₒTaₚM_{q}XᵣOₛ]^{y-},
wobei A mindestens ein Gegenion darstellt, wobei V, Mo, W, Nb, Ta und M Addenda-Atome sind, wobei M mindestens ein f- oder d-Blockelement mit mindestens einem d-Elektron außer Vanadium, Molybdän, Wolfram, Niob oder Tantal darstellt, wobei X mindestens ein Heteroatom ist, das aus p-, d- und f-Blockelementen außer Sauerstoff ausgewählt ist, wobei k im Bereich von 0 bis 30 liegt, wobei m im Bereich von 0 bis 160 liegt, wobei n im Bereich von 0 bis 160 liegt, wobei o im Bereich von 0 bis 30 liegt, wobei p im Bereich von 0 bis 10 liegt, wobei q im Bereich von 0 bis 30 liegt, wobei r im Bereich von 0 bis 30 liegt, wobei s ausreichend groß ist, dass y größer als null ist, wobei die Summe aus k, m, n, o und p gleich oder größer als vier ist und wobei ein oder mehrere der Sauerstoffatome in den Polyoxometallaten optional durch ein oder mehrere p-Blockelemente substituiert sein können,
derivatisierten und nicht derivatisierten Kohlenstoffnanofasern, derivatisierten und nicht derivatisierten Fullerenen, derivatisierten und nicht derivatisierten Keramiknanoröhrchen, derivatisierten und nicht derivatisierten Keramiknanofasern, derivatisierten und nicht derivatisierten Phyllosilikaten, derivatisierten und nicht derivatisierten polyedrischen oligomeren Silsequioxanen und deren Kombinationen.

9. Inneres Medizinprodukt nach Anspruch 1, wobei ein Therapeutikum auf dem oder in dem extrudierten Abschnitt vorgesehen ist, wobei ein Therapeutikum optional auf, unter oder in der Polymerschicht vorgesehen ist.

10. Medizinprodukt nach Anspruch 1, wobei mindestens ein Abschnitt des extrudierten Bereichs freistehend ist.

11. Medizinprodukt nach Anspruch 1, wobei das permanente darunter oder darüber liegende Substrat eine keramische, metallische oder polymerische Struktur ist, oder ein aufblasbares Ballonsubstrat aufweist, das unter dem extrudierten Bereich liegt.

12. Medizinprodukt nach Anspruch 1, wobei der extrudierte Abschnitt die Form einer Schicht hat, die die Gesamtheit oder einen Abschnitt eines darunter liegenden Medizinproduktsubstrats abdeckt.

13. Medizinprodukt nach Anspruch 7, wobei das Nanopartikel-Verstärkungsgebilde Nanopartikel aufweist, deren kleinstes Maß im Bereich von 0,5 bis 100 nm liegt.

## Revendications

1. Dispositif médical interne, le dispositif médical interne étant choisi parmi un greffon, un stent, un filtre et un ballonnet gonflable, comprenant une région extrudée qui comprend une partie extrudée, ladite partie extrudée comprenant un polyélectrolyte, au moins une partie de ladite région extrudée est disposée sur un substrat permanent sous-jacent ou superposé dudit dispositif médical interne et ledit dispositif médical interne étant adapté pour l'implantation ou l'insertion dans un sujet ; dans lequel la région extrudée est formée en utilisant une technique d'écriture directe, qui implique la formation de filaments à partir de fluides contenant un polyélectrolyte.

2. Dispositif médical interne selon la revendication 1, comprenant une pluralité de régions extrudées, ou
dans lequel ladite région extrudée comprend une pluralité desdites parties extrudées, dans lequel lesdites parties extrudées sont de préférence choisies parmi des parties extrudées pleines, des parties extrudées creuses et leurs combinaisons, ou
dans lequel ladite partie extrudée comprend une pluralité de polyélectrolytes, ou
dans lequel une couche polymère est prévue sur au moins une partie de ladite région extrudée.

3. Dispositif médical interne selon la revendication 1, dans lequel ladite partie extrudée comprend une espèce de polycation et une espèce de polyanion.

4. Dispositif médical interne selon la revendication 3, dans lequel lesdites espèces de polycation et de polyanion comprennent un rapport non stoechiométrique de groupes cationiques et anioniques ionisables, dans lequel lesdites espèces de polycation et de polyanion ont facultativement un poids moléculaire différent et dans lequel l'espèce de polycation et de polyanion de poids moléculaire supérieur est prévue en excès molaire par rapport à l'espèce de polycation ou de polyanion de poids moléculaire inférieur, ou
dans lequel ladite espèce de polyanion est un acide polyacrylique et dans lequel ladite espèce de polycation est choisie parmi la polyéthylénimine et le poly(hydrochlorure d'allylamine).

5. Dispositif médical interne selon la revendication 1, dans lequel ladite partie extrudée comprend une entité de renforcement.

6. Dispositif médical interne selon la revendication 5, dans lequel ladite entité de renforcement est une entité de renforcement dérivée du sol-gel.

7. Dispositif médical interne selon la revendication 5, dans lequel ladite entité de renforcement est une entité de renforcement nanoparticulaire.

8. Dispositif médical interne selon la revendication 7, dans lequel ladite entité de renforcement nanoparticulaire comprend des nanoparticules choisies parmi les nanotubes de carbone dérivés et non dérivés,
les polyoxométalates dérivés et non dérivés,
les polyoxométalates de la formule :
A[VₖMoₘWₙNbₒTaₚM_{q}XᵣO₅]^{y-},
dans laquelle A représente au moins un contre-ion, dans laquelle V, Mo, W, Nb, Ta et M sont des atomes addenda, dans laquelle M représente au moins un élément du bloc f ou d ayant au moins un électron d différent du vanadium, du molybdène, du tungstène, du niobium ou du tantale, dans laquelle X est au moins un hétéroatome choisi parmi les éléments des blocs p, d et f différents de l'oxygène, dans laquelle k est de l'ordre de 0 à 30, dans lequel m est de l'ordre de 0 à 160, dans lequel n est de l'ordre de 0 à 160, dans lequel o est de l'ordre de 0 à 30, dans lequel p est de l'ordre de 0 à 10, dans lequel q est de l'ordre de 0 à 30, dans lequel r est de l'ordre de 0 à 30, dans lequel s est suffisamment grand de sorte que y soit supérieur à zéro, dans laquelle la somme de k, m, n, o et p est supérieure ou égale à quatre, et dans laquelle un ou plusieurs atomes d'oxygène dans les polyoxométalates peuvent être facultativement remplacés par un ou plusieurs éléments du bloc p,
des nanofibres de carbone dérivées et non dérivées, des fullerènes dérivés ou non dérivés, des nanotubes de céramique dérivés et non dérivés, des nanofibres de céramique dérivées et non dérivées, des phyllosilicates dérivés et non dérivés, des silséquioxanes oligomères polyédriques dérivés et non dérivés, et leurs combinaisons.

9. Dispositif médical interne selon la revendication 1, dans lequel un agent thérapeutique est prévu sur ou à l'intérieur de ladite partie extrudée, dans lequel un agent thérapeutique est facultativement prévu sur, au-dessous ou à l'intérieur de ladite couche polymère.

10. Dispositif médical selon la revendication 1, dans lequel au moins une partie de ladite région extrudée est indépendante.

11. Dispositif médical selon la revendication 1, dans lequel ledit substrat permanent sous-jacent ou superposé est une structure en céramique, métallique ou polymère ou comprenant facultativement un substrat de ballonnet gonflable au-dessous de ladite région extrudée.

12. Dispositif médical selon la revendication 1, dans lequel ladite partie extrudée se présente sous la forme d'une couche qui recouvre la totalité ou une partie d'un substrat du dispositif médical sous-jacent.

13. Dispositif médical selon la revendication 7, dans lequel ladite entité de renforcement nanoparticulaire comprend des nanoparticules de l'ordre de 0,5 à 100 nm selon la plus petite dimension.
